# EUROPÄISCHE PATENTSCHRIFT

(11) **EP 3 412 207 B1**
(45) Veröffentlichungstag und Bekanntmachung des Hinweises auf die Patenterteilung: **01.04.2020**
(21) Anmeldenummer: 17206758.9
(22) Anmeldetag: 12.12.2017
(51) Int. Cl.: A61B 6/00, A61B 8/00

(54) **MAMMOGRAPHIE-BILDGEBUNG**
MAMMOGRAPHY IMAGING
IMAGERIE MAMMAIRE

(43) Veröffentlichungstag der Anmeldung: 12.12.2018
(73) Patentinhaber: Siemens Healthcare GmbH, 91052 Erlangen (DE)
(72) Erfinder: DÜPPENBECKER, Peter Michael, 52070 Aachen (DE); RADICKE, Marcus, 90587 Veitsbronn (DE); SCHMIDT, Oliver, 91052 Erlangen (DE)

(56) Entgegenhaltungen:
- DE-A1- 10 255 856
- DE-A1-102013 219 252
- DE-A1-102015 218 607
- JP-A- 2008 173 291
- US-A- 5 664 573

## Beschreibung

Die Erfindung betrifft eine Mammographieanlage zur röntgenographischen und sonographischen Bildgebung einer Brust einer Patientin, sowie ein Verfahren für ein Bereitstellen eines röntgenographischen Bilddatensatzes und eines sonographischen Bilddatensatzes der Brust der Patientin.

Üblicherweise kommt zum Screening und zur Diagnose von Brustkrebs vorwiegend eine röntgenbasierte Mammographieanlage zum Einsatz, womit eine röntgenographische Bildgebung einer Brust einer Patientin durchgeführt wird. Insbesondere junge Frauen weisen einen hohen Anteil von dichtem Brustgewebe auf. Das dichte Brustgewebe absorbiert Röntgenstrahlung bei der röntgenographischen Bildgebung in etwa wie potentielles Tumorgewebe, wodurch eine Differenzierung zwischen dichtem Brustgewebe und potentiellem Tumorgewebe erschwert wird.

Einer Patientin mit dichtem Brustgewebe wird daher vorzugsweise zusätzlich eine sonographische Bildgebung der Brust empfohlen. Insbesondere der zur Röntgenabsorption komplementäre Kontrast der sonographischen Bildgebung kann eine sichere Diagnose ermöglichen. Die sonographische Bildgebung kann in diesem Fall beispielsweise entweder händisch mit einem herkömmlichen Ultraschallkopf oder mittels eines automatischen Brust-Volumen-Scanners durchgeführt werden. Die händische Bildgebung ist üblicherweise relativ zeitintensiv und erfordert insbesondere einen erfahrenen Arzt oder Sonographer. Der automatische Brust-Volumen-Scanner weist typischerweise einen linearen Ultraschallwandler und eine mechanische Verfahreinheit auf, wodurch die sonographische Bildgebung der Brust der Patientin automatisch, insbesondere ohne Eingriff des Arztes, durchgeführt werden kann. Eine derartige Untersuchung der Brust kann jedoch bis zu 20 Minuten dauern. Daher werden üblicherweise für die röntgenographische Bildgebung und die sonographische Bildgebung zwei separate Termine vereinbart.

Während die röntgenographische Bildgebung der komprimierten Brust typischerweise in einer stehenden Pose der Patientin durchgeführt wird, liegt die Patientin üblicherweise bei der sonographischen Bildgebung, bei welcher die Brust typischerweise nur gering oder gar nicht komprimiert ist. Ein röntgenographischer Bilddatensatz der röntgenographischen Bildgebung und ein sonographischer Bilddatensatz der sonographischen Bildgebung sind daher üblicherweise schwer von einem Radiologen parallel zu begutachten. Üblicherweise ist allerdings eine exakte Bildregistrierung des röntgenographischen Bilddatensatzes und des sonographischen Bilddatensatzes nicht möglich.

Die DE 10 2008 009 967 A1 offenbart eine Mammographieanlage zur Untersuchung einer Brust, wobei zwischen der röntgenographischen Untersuchung und der sonographischen Untersuchung eine erste Lagerplatte und eine zweite, einen Ultraschallwandler umfassende Lagerplatte ausgetauscht werden.

Die DE 10 2013 219 252 offenbart ebenfalls eine Mammographieanlage zur Untersuchung der Brust, umfassend einen flächig ausgebildeten Ultraschallwandler, der in eine Kompressionsvorrichtung integriert ist.

Der Erfindung liegt die Aufgabe zu Grunde, eine Mammographieanlage anzugeben, welche eine röntgenographische und sonographische Bildgebung in unmittelbarer Abfolge ermöglicht.

Die Aufgabe wird durch die Merkmale der unabhängigen Ansprüche gelöst. Vorteilhafte Weiterbildungen sind in den abhängigen Ansprüchen angegeben.

Die erfindungsgemäße Mammographieanlage zur röntgenographischen und sonographischen Bildgebung einer Brust einer Patientin weist
- eine erste Kompressionsvorrichtung,
- eine zweite Kompressionsvorrichtung,
- eine Stützvorrichtung,
- einen Röntgenstrahler,
- einen integrierten Röntgendetektor und
- einen integrierten Ultraschallwandler auf, welcher flächig ausgebildet ist,
- wobei die zweite Kompressionsvorrichtung den integrierten Röntgendetektor aufweist,
- wobei die Stützvorrichtung an der ersten Kompressionsvorrichtung oder an der zweiten Kompressionsvorrichtung angeordnet ist und
- wobei zwischen der Stützvorrichtung und derjenigen Kompressionsvorrichtung, die der Kompressionsvorrichtung, an welcher die Stützvorrichtung angeordnet ist, gegenüberliegt, ein Brustaufnahmebereich vorgesehen ist und die Brust der Patientin im Brustaufnahmebereich positionierbar ist,
- wobei die Stützvorrichtung den integrierten Ultraschallwandler aufweist und
- wobei der integrierte Ultraschallwandler in Richtung des Brustaufnahmebereichs ausgerichtet ist,
dadurch gekennzeichnet,
dass die Stützvorrichtung und der integrierte Ultraschallwandler derart deformierbar ausgebildet sind, dass sie an die Brust der Patientin anformbar sind, wobei der integrierte Ultraschallwandler auf einem elastischen Substrat angeordnet ist.

Die Mammographieanlage ist insbesondere zur röntgenographischen und sonographischen Bildgebung einer Brust eines Patienten unabhängig von einem Geschlecht des Patienten geeignet. Der Begriff Patientin umfasst insbesondere auch einen männlichen Patienten. Die Mammographieanlage weist typischerweise ein Stativ auf, wobei mit dem Stativ insbesondere die erste Kompressionsvorrichtung, die zweite Kompressionsvorrichtung und der Röntgenstrahler typischerweise verschiebbar verbunden sind. Die Mammographieanlage kann insbesondere eine Systemachse aufweisen, wobei eine Körperachse der Patientin typischerweise annähernd parallel zu der Systemachse während der röntgenographischen Bildgebung und der sonographischen Bildgebung ausgerichtet ist. Üblicherweise kann der Röntgenstrahler, die erste Kompressionsvorrichtung, die zweite Kompressionsvorrichtung, die Stützvorrichtung und/oder der integrierte Röntgendetektor um eine Drehachse bis zu einem Drehwinkel von 90° verdreht werden. Die Drehachse steht insbesondere senkrecht auf der Systemachse der Mammographieanlage. Beispielsweise kann durch das Verdrehen der ersten Kompressionsvorrichtung und der zweiten Kompressionsvorrichtung die Brust der Patientin aus unterschiedlichen Winkeln komprimiert werden, wodurch die sonographische Bildgebung und die röntgenographische Bildgebung verschiedene Winkel, insbesondere verschiedene Ansichten, der Brust der Patientin erfassen kann. Wenn der Drehwinkel 0° beträgt und die Körperachse annährend parallel zur Systemachse ausgerichtet ist, wird eine derartige Bildgebung cranio-caudal genannt.

Typischerweise ist die Mammographieanlage für eine röntgenographische Bildgebung und sonographische Bildgebung in stehender Pose der Patientin ausgerichtet. Derart sind in einem typischen Anwendungsfall, insbesondere wenn der Drehwinkel 0° beträgt, die erste Kompressionsvorrichtung und die zweite Kompressionsvorrichtung vertikal übereinanderliegend angeordnet, wodurch je nach Anordnung die erste Kompressionsvorrichtung und die zweite Kompressionsvorrichtung als obere bzw. untere Kompressionsvorrichtung bezeichnet werden können.

Der Röntgenstrahler und der integrierte Röntgendetektor bilden insbesondere ein Röntgensystem, welches zu der röntgenographischen Bildgebung ausgebildet ist. Das Röntgensystem weist typischerweise eine röntgenographische Steuereinheit auf, welche die röntgenographische Bildgebung mittels des Röntgensystems steuert und/oder insbesondere zu einer Rekonstruktion eines röntgenographischen Bilddatensatzes gemäß der röntgenographischen Bildgebung ausgebildet ist. Der Röntgenstrahler ist vorzugsweise auf den integrierten Röntgendetektor ausgerichtet. Das Röntgensystem weist typischerweise einen Strahlengang und ein maximales Gesichtsfeld auf, wobei typischerweise der Strahlengang größer als das maximale Gesichtsfeld ist sowie das maximale Gesichtsfeld vorzugsweise vollständig umschließt. Der Strahlengang beschreibt insbesondere zumindest einen Pfad für Röntgenstrahlen ausgehend von dem Röntgenstrahler zum integrierten Röntgendetektor. Das maximale Gesichtsfeld weist insbesondere einen Messbereich der röntgenographischen Bildgebung auf. Typischerweise ist zwischen dem Röntgenstrahler und der zweiten den integrierten Röntgendetektor aufweisenden Kompressionsvorrichtung die erste Kompressionsvorrichtung angeordnet. Die Röntgenstrahlen werden in diesem Fall beispielsweise durch die erste Kompressionsvorrichtung und die den integrierten Ultraschallwandler aufweisende Stützvorrichtung gedämpft bzw. absorbiert. Die erste Kompressionsvorrichtung und die Stützvorrichtung sowie typischerweise die zweite Kompressionsvorrichtung sind daher vorzugsweise besonders röntgentransparent ausgebildet, wodurch insbesondere die Dämpfung bzw. die Absorption der Röntgenstrahlen verringert ist.

Grundsätzlich ist es denkbar, dass der Röntgenstrahler parallel zu der ersten Kompressionsvorrichtung und/oder der zweiten Kompressionsvorrichtung linear verschiebbar angeordnet ist. Alternativ oder zusätzlich kann der Röntgenstrahler auf einem Kreisbogen verschiebbar angeordnet sein, wobei der Kreisbogen um eine Drehachse definiert ist, welche senkrecht zur Systemachse der Mammographieanlage ausgerichtet ist. In diesem Fall kann beispielsweise eine dreidimensionale röntgenographische Bildgebung, insbesondere eine röntgenographische Tomosynthese, mittels der Mammographieanlage durchgeführt werden. Im Allgemeinen kann bei einer Tomosynthese ein dreidimensionales Bild aus zweidimensionalen Bildern, welche aus verschiedenen Winkeln des Röntgenstrahlers relativ zum Röntgendetektor erfasst werden, erzeugt werden. Die zweidimensionalen und/oder dreidimensionalen Bilder können Teil des sonographischen bzw. röntgenographischen Bilddatensatzes sein.

Typischerweise kann ein Abstand zwischen der ersten Kompressionsvorrichtung und der zweiten Kompressionsvorrichtung verändert werden, insbesondere zu einer Kompression der Brust. Grundsätzlich ist es denkbar, dass eine der beiden Kompressionsvorrichtungen die Brust auf die andere Kompressionsvorrichtung drückt. Sprich eine der beiden Kompressionsvorrichtungen ist verschiebbar, wohingegen die andere Kompressionsvorrichtung starr befestigt ist. Typischerweise wird die Brust der Patienten dadurch komprimiert, dass der Abstand zwischen der ersten Kompressionsvorrichtung und der zweiten Kompressionsvorrichtung verringert wird. Die erste Kompressionsvorrichtung und/oder die zweite Kompressionsvorrichtung sind daher insbesondere entlang der Systemachse relativ zueinander sowie relativ zu der Brust der Patientin verschiebbar, im Fall einer vertikalen Anordnung der Mammographieanlage also insbesondere höhenverstellbar, ausgebildet. Weiterhin sind die erste Kompressionsvorrichtung und die zweite Kompressionsvorrichtung üblicherweise derart ausgebildet, dass jeweils eine flache Seite der ersten Kompressionsvorrichtung sowie der zweiten Kompressionsvorrichtung aufeinander, insbesondere parallel zueinander, ausgerichtet ist. Wenn die Brust der Patientin im Brustaufnahmebereich, beispielsweise auf der flachen Seite der unteren Kompressionsvorrichtung lagernd, positioniert ist, wird die Brust insbesondere komprimiert durch ein derartiges Einstellen der ersten Kompressionsvorrichtung, insbesondere der oberen Kompressionsvorrichtung, und der zweiten Kompressionsvorrichtung, dass der Abstand zwischen der ersten Kompressionsvorrichtung und der zweiten Kompressionsvorrichtung verändert, insbesondere verringert wird. Die erste Kompressionsvorrichtung bzw. die zweite Kompressionsvorrichtung können jeweils als Kompressionsplatte ausgebildet sein.

Die erste Kompressionsvorrichtung und/oder die zweite Kompressionsvorrichtung können jeweils eine Aufnahmevorrichtung für die Stützvorrichtung aufweisen. Die Stützvorrichtung kann somit insbesondere in der Aufnahmevorrichtung der ersten Kompressionsvorrichtung und/oder der Aufnahmevorrichtung der zweiten Kompressionsvorrichtung angeordnet sein. Die Stützvorrichtung ist insbesondere zu einem Stützen und/oder zu einem Fixieren der Brust ausgebildet. In anderen Worten wird die Brust insbesondere mittels der Stützvorrichtung in einer Position gehalten. Die Stützvorrichtung ist insbesondere zwischen der ersten Kompressionsvorrichtung und der zweiten Kompressionsvorrichtung angeordnet. Typischerweise wird diejenige Kompressionsvorrichtung, an welcher die Stützvorrichtung angeordnet ist, als Kompressionsvorrichtung mit der Stützvorrichtung oder stützende Kompressionsvorrichtung und die gegenüberliegende Kompressionsvorrichtung als freie Kompressionsvorrichtung bezeichnet. Die Stützvorrichtung ist typischerweise derart angeordnet, dass die Stützvorrichtung an der stützenden Kompressionsvorrichtung, insbesondere lösbar, befestigt ist. Beispielsweise ist die Stützvorrichtung während der sonographischen Bildgebung und der röntgenographischen Bildgebung mit der stützenden Kompressionsvorrichtung fest verbunden und kann danach beispielsweise zu einer Reinigung der Stützvorrichtung abgenommen und später wiederum angeordnet werden.

Der integrierte Ultraschwallwandler ist flächig, also insbesondere in dem Sinne zweidimensional ausgebildet, dass eine Ausdehnung in einer dritten Dimension vorzugsweise vernachlässigbar ist. Der integrierte Ultraschallwandler, insbesondere eine Seite des integrierten Ultraschallwandlers, bildet üblicherweise eine breitere Fläche, dehnt sich auf einer Fläche aus und/oder ist abgeflacht. Der integrierte Ultraschallwandler kann starr und/oder eben ausgebildet sein. In anderen Worten kann der integrierte Ultraschallwandler einen planaren Zustand, beispielsweise in einer Ausgangsposition, aufweisen, wobei der integrierte Ultraschallwandler beim planaren Zustand nicht deformiert ist. Wenn der integrierte Ultraschallwandler an die Brust angeformt ist, weist der integrierte Ultraschallwandler typischerweise einen deformierten Zustand auf. Unter der Annahme, dass die Systemachse parallel zur z-Achse der Raumkoordinaten ausgerichtet ist, ist der integrierte Ultraschallwandler insbesondere in der x-y-Ebene flächig ausgebildet. Es ist denkbar, dass eine Seite des integrierten Ultraschallwandlers flächig ausgebildet ist. Der integrierte Ultraschallwandler kann üblicherweise scheibenförmig, quaderförmig oder würfelförmig ausgebildet sein, wobei in diesen Fällen zumindest eine Seite des integrierten Ultraschallwandlers flächig ausgebildet ist. Beispielsweise kann die flächige Seite des integrierten Ultraschallwandlers eine Kantenlänge aufweisen, welche zweimal, vorzugsweise fünfmal, besonders bevorzugt zehnmal länger ist als eine Kantenlänge des integrierten Ultraschallwandlers senkrecht zur flächigen Seite. Der integrierte Ultraschallwandler ist vorzugsweise derart ausgebildet, dass dieser insbesondere im Vergleich zu einem herkömmlichen Ultraschallkopf auf einer größeren Fläche, vorzugsweise gleichzeitig, mit der Brust der Patientin in Kontakt stehen kann. In anderen Worten ist vorzugsweise eine akustische Kopplung des integrierten Ultraschallwandlers zur Brust der Patientin größer als im Vergleich zu dem herkömmlichen Ultraschallkopf.

Alternativ oder zusätzlich kann der integrierte Ultraschallwandler derart ausgebildet sein, dass eine Seite des integrierten Ultraschallwandlers eine flächige Ausdehnung aufweist. Der integrierte Ultraschallwandler weist insbesondere eine Vielzahl von Ultraschallwandlerelementen auf, die vorzugsweise in einer flächigen Matrix angeordnet sind. Der integrierte Ultraschallwandler kann insbesondere zu dem Stützen bzw. dem Fixieren der Brust ausgebildet sein. Der integrierte Ultraschallwandler basiert vorzugsweise auf einer pMUT-Technologie (piezoelectric micromachined ultrasound transducer). Die pMUT-Technologie ermöglicht insbesondere eine Herstellung des flächigen, integrierten Ultraschallwandlers bei vorzugsweise geringen Kosten. Ein weiterer Vorteil des integrierten Ultraschallwandlers basierend auf pMUT-Technologie ist, dass typischerweise solche Materialien zum Aufbau der Ultraschallwandlerelemente gewählt werden, welche zumindest teilweise röntgentransparent sind. Insbesondere im Vergleich zu einer cMUT-Technologie (capacitive microfabricated ultrasound transducer) und zu einer PZT-Technologie (Piezo-Zirkonat-Citanat) weist die pMUT-Technologie eine höhere Röntgentransparenz auf. In anderen Worten ist der integrierte Ultraschallwandler vorzugsweise röntgentransparent ausgebildet. Die Röntgentransparenz ist insbesondere vorteilhaft, weil dadurch der integrierte Ultraschallwandler während der röntgenographischen Bildgebung in dem Strahlengang des Röntgensystems verbleiben kann. Vorzugsweise beträgt die Röntgentransparenz des integrierten Ultraschallwandlers bei einer für die röntgenographische Bildgebung typischen Röntgenenergie von 28 keV mindestens 30%, vorzugsweise mehr als 50% oder besonders bevorzugt mehr als 80%. Die Röntgenenergie beschreibt insbesondere eine maximale Röntgenenergie der Röntgenstrahlen beim Verlassen des Röntgenstrahlers und wird insbesondere von der röntgenographischen Steuereinheit vorgegeben bzw. geregelt. In anderen Worten können bei der typischen Röntgenenergie von 28 keV und der 30%-Röntgentransparenz beispielsweise näherungsweise 30% aller Röntgenstrahlen den integrierten Ultraschallwandler durchdringen.

Der integrierte Ultraschallwandler ist insbesondere Teil eines Ultraschallsystems, welches eine sonographische Steuereinheit aufweisen kann, wobei die sonographische Steuereinheit insbesondere die sonographische Bildgebung steuert und/oder insbesondere zu einer Rekonstruktion eines sonographischen Bilddatensatzes gemäß der sonographischen Bildgebung ausgebildet ist. Alternativ oder zusätzlich kann die Mammographieanlage eine Steuereinheit aufweisen, wobei die Steuereinheit der Mammographieanlage die röntgenographische Steuereinheit und die sonographische Steuereinheit aufweist. Die sonographische Steuereinheit gibt vorzugsweise eine sonographische Messsequenz sowie eine Frequenz von Ultraschallwellen ausgehend vom integrierten Ultraschallwandler gemäß der sonographischen Messsequenz vor. Typischerweise definiert die Frequenz der Ultraschallwellen ein maximales Gesichtsfeld des integrierten Ultraschallwandlers, insbesondere eine maximale Eindringtriefe der Ultraschallwellen, wobei das maximale Gesichtsfeld insbesondere einen Messbereich der sonographischen Bildgebung aufweist. Der Messbereich der sonographischen Bildgebung ist demnach im Wesentlichen durch eine geometrische Größe der flächig ausgebildeten Seite des integrierten Ultraschallwandlers und der Frequenz der Ultraschallwellen vorgegeben. Vorzugsweise ist eine Schnittmenge des Messbereichs der sonographischen Bildgebung und des Messbereichs der röntgenographischen Bildgebung ungleich Null. Insbesondere ist die Schnittmenge größer als ein außerhalb der Schnittmenge liegender Bereich des Messbereichs der sonographischen Bildgebung und/oder des Messbereichs der röntgenographischen Bildgebung. Üblicherweise beträgt die Schnittmenge zwischen dem Messbereich der sonographischen Bildgebung und dem Messbereich der röntgenographischen Bildgebung mehr als 20%, vorzugsweise mehr als 50% oder besonders bevorzugt mindestens 80%. Vorzugsweise umfasst der Messbereich der sonographischen Bildgebung oder der Messbereich der röntgenographischen Bildgebung den jeweils anderen Messbereich vollständig. In anderen Worten ist vorzugsweise der Messbereich der sonographischen Bildgebung oder der Messbereich der röntgenographischen Bildgebung ein vollständiger Teilbereich des anderen Messbereichs. Besonders bevorzugt ist eine Ausführung, bei der der Messbereich der sonographischen Bildgebung und der Messbereich der röntgenographischen Bildgebung deckungsgleich sind.

Der integrierte Ultraschwallwandler sendet insbesondere die Ultraschallwellen in Richtung des Brustaufnahmebereichs aus, wodurch die sonographische Bildgebung im Brustaufnahmebereich ermöglicht wird. So wird insbesondere die sonographische Bildgebung der Brust der Patientin ermöglicht, wenn die Brust der Patientin derart im Brustaufnahmebereich positioniert ist, dass die akustische Kopplung zwischen dem integrierten Ultraschallwandler und der Brust der Patientin vorhanden ist. Die akustische Kopplung kann beispielsweise mittels einer Verwendung eines Ultraschallgels und/oder mittels der Kompression der Brust erhöht werden. Typischerweise ist der integrierte Ultraschallwandler zusätzlich zu einem Empfangen der Ultraschallwellen ausgebildet. Grundsätzlich ist auch denkbar, dass der integrierte Ultraschallwandler lediglich zum Senden oder zum Empfangen der Ultraschallwellen ausgebildet ist. Vorzugsweise kann zwischen Senden oder Empfangen sowie Senden und Empfangen mittels der sonographischen Steuereinheit umgeschaltet werden.

Der Brustaufnahmebereich ist typischerweise zwischen der Stützvorrichtung und der freien Kompressionsvorrichtung vorgesehen. Der Brustaufnahmebereich umfasst insbesondere eine Ausdehnung zwischen der Stützvorrichtung und der freien Kompressionsvorrichtung entlang der Systemachse. In anderen Worten ist der Brustaufnahmebereich typischerweise vorgegeben durch den Abstand zwischen der ersten Kompressionsvorrichtung sowie der zweiten Kompressionsvorrichtung, wobei von diesem Abstand insbesondere eine Ausdehnung der Stützvorrichtung entlang der Systemachse abgezogen wird. Der Brustaufnahmebereich ermöglicht vorzugsweise die Positionierung der Brust der Patientin, insbesondere im Messbereich der sonographischen Bildgebung und/oder im Messbereich der röntgenographischen Bildgebung. Derart ist die Brust der Patientin zwischen der Stützvorrichtung und der freien Kompressionsvorrichtung positionierbar. Eine Schnittmenge des Brustaufnahmebereichs, des Messbereichs der sonographischen Bildgebung und des Messbereichs der röntgenographischen Bildgebung ist typischerweise ungleich Null. Üblicherweise ist jeweils ein Teilbereich des Brustaufnahmebereichs, des Messbereichs der sonographischen Bildgebung und des Messbereichs der röntgenographischen Bildgebung in den jeweils anderen Bereichen enthalten. Besonders bevorzugt ist, wenn der Brustaufnahmebereich vollständig im Messbereich der sonographischen Bildgebung und im Messbereich der röntgenographischen Bildgebung enthalten ist.

Ein Vorteil der Mammographieanlage ist insbesondere, dass der integrierte Ultraschallwandler während der röntgenographischen Bildgebung im Strahlengang des Röntgensystems verbleiben kann. In anderen Worten ist der integrierte Ultraschallwandler vorzugsweise derart ausgebildet, dass die röntgenographische Bildgebung möglich ist, obwohl der integrierte Ultraschwallwandler zwischen dem Röntgenstrahler und dem Röntgendetektor angeordnet ist. Besonders vorteilhaft ist eine röntgentransparente Ausgestaltung des integrierten Ultraschallwandlers. Ein weiterer Vorteil ist üblicherweise, dass aufgrund der flächigen Ausbildung des integrierten Ultraschallwandlers der Messbereich der sonographischen Bildgebung den für die Diagnose relevanten Bereich der Brust der Patientin bis zu 70% abdeckt. Typischerweise ist keine mechanische Verfahreinheit für die sonographische Bildgebung nötig. Besonders vorteilhaft ist, dass daher die sonographische Bildgebung und die röntgenographische Bildgebung quasi unmittelbar nacheinander, in einer beliebigen Reihenfolge erfolgen können, wodurch eine Gesamtdauer der sonographischen Bildgebung und der röntgenographischen Bildgebung verringert und die potentiell für die Patientin schmerzhafte Kompression der Brust verkürzt ist. Ein weiterer Vorteil ist insbesondere, dass der röntgenographische Bilddatensatz und der sonographische Bilddatensatz insbesondere deckungsgleich sind, sprich den gleichen Bereich der Brust der Patientin abbilden, wodurch typischerweise die Diagnose oder eine Befundung verbessert werden kann. In anderen Worten ist vorzugsweise eine Bildregistrierung zwischen dem röntgenographischen Bilddatensatz und dem sonographischen Bilddatensatz überhaupt, insbesondere exakt, möglich bzw. in einem besonders bevorzugten Fall nicht nötig.

Die Stützvorrichtung und der integrierte Ultraschallwandler sind derart deformierbar ausgebildet, dass sie an die Brust der Patientin anformbar sind, wobei der integrierte Ultraschallwandler auf dem elastischen Substrat angeordnet ist. Insbesondere die pMUT-Technologie ermöglicht eine technische Realisierung des deformierbaren, integrierten Ultraschallwandlers. Üblicherweise kann sich der deformierbare, integrierte Ultraschwallwandler flächig an die Brust der Patientin anformen, wodurch vorzugsweise die akustische Ankopplung der Brust an den integrierten Ultraschallwandler verbessert wird. Ein Vorteil kann sein, dass aufgrund der deformierbaren Ausbildung des integrierten Ultraschallwandlers der Messbereich der sonographischen Bildgebung den für die Diagnose relevanten Bereich der Brust der Patientin zu mehr als 70%, besonders bevorzugt zu mehr als 95% abdeckt.

Die Ultraschallwandlerelemente liegen insbesondere in einem deformierten, insbesondere gekrümmten Zustand des integrierten Ultraschallwandlers, auf einer Oberfläche der Brust der Patientin auf. Der integrierte Ultraschallwandler kann sich vorzugsweise also der Oberfläche der Brust der Patientin anpassen und/oder die Oberfläche der Brust der Patientin bis zu einem gewissen Grad verformen. Der integrierte Ultraschallwandler ist vorzugsweise derart auf dem elastischen Substrat angeordnet, dass der integrierte Ultraschallwandler in alle Raumrichtungen an die Brust angeformt werden kann. Typischerweise kann der Ultraschallwandler entlang einer Achse von einer Pectoralis der Patientin zu einer Mamille der Patientin, und/oder entlang einer Achse, welche parallel zur Pectoralis der Patientin verläuft gekrümmt werden, um insbesondere die Form der Brust abzubilden. Im Vergleich zu einem undeformierbaren Ultraschallwandler besteht der Vorteil, dass der deformierbare, integrierte Ultraschallwandler an die konkave und individuelle Form der Brust der Patientin angepasst werden kann. Des Weiteren kann insbesondere in einem Randbereich der Brust der Patientin auf zusätzliches Ultraschallgel verzichtet werden, wodurch vorzugsweise die Absorption der Röntgenstrahlen in dem Ultraschallgel verringert wird.

Eine Ausführungsform sieht vor, dass die Stützvorrichtung eine Membran aufweist, wobei an der Membran der integrierte Ultraschallwandler angeordnet ist. Die Membran kann beispielsweise ein Textil, Glas, Folie, Gewebe und/oder Gaze aufweisen. Grundsätzlich ist es denkbar, dass die Membran den integrierten Ultraschallwandler zumindest teilweise umfasst oder dass der integrierte Ultraschallwandler innerhalb der Membran angeordnet ist. Ein Vorteil dieser Ausführungsform ist, dass die Stützvorrichtung vorzugsweise an die Brust der Patientin anformbar ist.

Eine Ausführungsform sieht vor, dass diejenige Kompressionsvorrichtung, an welcher die Stützvorrichtung angeordnet ist, als ein Rahmen ausgebildet ist und die Stützvorrichtung innerhalb des Rahmens eingespannt ist. Insbesondere wenn die Stützvorrichtung die Membran aufweist, ist vorzugsweise die Membran innerhalb des Rahmens eingespannt. Die Stützvorrichtung, insbesondere der integrierte Ultraschallwandler, ist vorzugsweise innerhalb des Rahmens befestigt. Der Rahmen ist insbesondere O-förmig, U-förmig, C-förmig oder rechteckig mit einer zentralen Aussparung ausgebildet.

Eine Ausführungsform sieht vor, dass die Mammographieanlage eine Recheneinheit aufweist, wobei die Mammographieanlage zu einem Ermitteln einer Deformation des integrierten Ultraschallwandlers ausgebildet ist und die Recheneinheit zu einem Rekonstruieren eines sonographischen Bilddatensatzes gemäß der Deformation ausgebildet ist. Insbesondere wenn die Steuereinheit der Mammographieanlage, insbesondere die röntgenographische Steuereinheit und/oder die sonographische Steuereinheit, in Programmcodemitteln abgebildet sind, kann vorzugsweise die Recheneinheit die Programmcodemittel ausführen. Grundsätzlich ist es denkbar, dass die Mammographieanlage eine sonographische Recheneinheit zur sonographischen Bildgebung und/oder eine röntgenographische Recheneinheit zur röntgenographischen Bildgebung aufweist. Das Ermitteln der Deformation wird in einem der folgenden Abschnitte beschrieben.

Eine Ausführungsform sieht vor, dass die Stützvorrichtung ein Kissen aufweist. Das Kissen ist vorzugsweise als weiche Unterlage bzw. als Polster für die Brust der Patientin ausgebildet. Typischerweise ist das Kissen an die Brust der Patientin anformbar. Das Kissen erhöht üblicherweise aufgrund seiner Beschaffenheit einen Komfort der Patientin bei der sonographischen Bildgebung und der röntgenographischen Bildgebung.

Eine Ausführungsform sieht vor, dass die Stützvorrichtung eine dem Brustaufnahmebereich zugewandte erste Seite und eine derjenigen Kompressionsvorrichtung, an der die Stützvorrichtung angeordnet ist, zugewandte zweite Seite aufweist, wobei die erste Seite den integrierten Ultraschallwandler aufweist. Der Vorteil dieser Ausführungsform ist, dass der integrierte Ultraschallwandler vorzugsweise unmittelbar mit der Brust der Patientin in Kontakt steht, wodurch die für die sonographische Bildgebung akustische Ankopplung gewährleistet ist. In diesem Fall ist also zwischen den Ultraschallwandlerelementen und der Brust des Patienten vorzugsweise lediglich Ultraschallgel, wodurch die akustische Ankopplung typischerweise verbessert wird.

Eine Ausführungsform sieht vor, dass die zweite Seite der Stützvorrichtung das Kissen aufweist. In anderen Worten ist vorzugsweise das Kissen zwischen dem integrierten Ultraschallwandler und der stützenden Kompressionsvorrichtung angeordnet. In diesem Fall ist also vorzugsweise der integrierte Ultraschallwandler in Kontakt mit der Brust der Patientin und zwischen dem integrierten Ultraschallwandler und der stützenden Kompressionsvorrichtung befindet sich insbesondere das Kissen. Insbesondere kann das Kissen ein Luftkissen oder ein Ultraschallgelkissen sein, weil das Kissen auf der den Ultraschallwellen abgewandten Seite angeordnet ist und vorzugsweise daher nicht die akustische Ankopplung beeinflusst. Grundsätzlich ist es denkbar, dass der integrierte Ultraschallwandler als Teil einer Hülle des Kissens ausgebildet ist. In diesem Fall ist vorzugsweise eine an die Brust der Patientin angepasste sonographische Bildgebung möglich, beispielsweise weil der integrierte Ultraschallwandler deformierbar und/oder das Kissen an sich deformierbar ausgebildet ist.

Eine Ausführungsform sieht vor, dass die Stützvorrichtung eine dem Brustaufnahmebereich zugewandte erste Seite und eine derjenigen Kompressionsvorrichtung, an der die Stützvorrichtung angeordnet ist, zugewandte zweite Seite aufweist, wobei die zweite Seite den integrierten Ultraschallwandler aufweist. Diese Ausführungsform grenzt sich zu der oben beschriebenen Ausführungsform insbesondere über eine Anordnung des integrierten Ultraschallwandlers in Bezug auf die Stützvorrichtung ab. Der Vorteil dieser Ausführungsform ist, dass der integrierte Ultraschallwandler insbesondere starr, sprich planar, ausgebildet sein kann. In diesem Fall ist beispielsweise die Stützvorrichtung an die Brust der Patientin anformbar.

Eine Ausführungsform sieht vor, dass die erste Seite das Kissen aufweist. In anderen Worten ist das Kissen vorzugsweise zwischen dem integrierten Ultraschallwandler und dem Brustaufnahmebereich angeordnet. In diesem Fall kann der integrierte Ultraschallwandler deformierbar oder starr ausgebildet sein, weil vorzugsweise das Kissen, beispielsweise als Ultraschallgelkissen, derart ausgebildet ist, dass das Kissen in Kombination mit dem Ultraschallgel die akustische Kopplung gewährleistet und vorzugsweise das Kissen an die Brust der Patientin anformbar ist.

Eine Ausführungsform sieht vor, dass die Mammographieanlage eine Recheneinheit aufweist, wobei der integrierte Ultraschallwandler zu einem Erfassen einer Deformation der Stützvorrichtung ausgebildet ist und die Recheneinheit zu einem Rekonstruieren eines röntgenographischen Bilddatensatzes gemäß der Deformation der Stützvorrichtung ausgebildet ist. Das Erfassen der Deformation der Stützvorrichtung kann insbesondere das Erfassen einer Ausdehnung, insbesondere eines Volumens, und/oder einer Position des Ultraschallgelkissens und/oder des Ultraschallgels mittels der sonographischen Bildgebung umfassen. Vorzugsweise kann gemäß dem sonographischen Bilddatensatz das Volumen des Ultraschallgelkissens bzw. des Ultraschallgels ermittelt werden, wobei dieses Volumen zu einem Anpassen des röntgenographischen Bilddatensatz vorzugsweise verwendet wird. Das Anpassen des röntgenographischen Bilddatensatz kann insbesondere eine Korrektur der Absorption der Röntgenstrahlen, abgebildet insbesondere in dem röntgenographischen Bilddatensatz, in Bezug auf das Volumen des Ultraschallgelkissens bzw. des Ultraschallgels umfassen. Insbesondere können Streustrahlen der röntgenographischen Bildgebung erfasst bzw. abgeschätzt werden und der röntgenographische Bilddatensatz kann gemäß der Streustrahlen korrigiert werden. Ein Vorteil dieser Ausführungsform ist daher, dass der sonographische Bilddatensatz für eine Verbesserung einer Bildqualität des röntgenographischen Bilddatensatzes verwendet kann.

Eine Ausführungsform sieht vor, dass die Mammographieanlage zusätzlich zu dem integrierten Ultraschallwandler einen weiteren Ultraschwallwandler aufweist, wobei der weitere Ultraschallwandler derart angeordnet ist, dass der Brustaufnahmebereich zwischen dem integrierten Ultraschallwandler und dem weiteren Ultraschallwandler vorgesehen ist. Vorzugsweise ist die sonographische Steuereinheit derart ausgebildet, dass der integrierte Ultraschallwandler die Ultraschallwellen empfangen kann, wenn der weitere Ultraschallwandler die Ultraschallwellen sendet, oder umgekehrt. Beispielsweise weisen die erste Kompressionsvorrichtung und die zweite Kompressionsvorrichtung jeweils einen Ultraschallwandler auf. In diesem Fall kann beispielsweise eine weitere Stützvorrichtung den weiteren Ultraschallwandler aufweisen, wobei der weitere Ultraschallwandler zwischen der ersten Kompressionsvorrichtung und der zweiten Kompressionsvorrichtung angeordnet ist. Alternativ kann der weitere Ultraschallwandler außerhalb der ersten Kompressionsvorrichtung und der zweiten Kompressionsvorrichtung angeordnet sein. Grundsätzlich ist auch denkbar, dass der weitere Ultraschallwandler zwischen der zweiten Kompressionsvorrichtung und dem integrierten Röntgendetektor angeordnet ist. Der weitere Ultraschallwandler kann im Vergleich zum integrierten Ultraschallwandler leistungsfähiger und größer sein sowie beispielsweise gemäß der PZT-Technologie ausgebildet sein. Diese Ausführungsformen können insbesondere eine sonographische Transmission-Bildgebung und/oder die sonographische Bildgebung mit einer höheren Frequenz der Ultraschallwellen ermöglichen. Die Mammographieanlage ist vorzugsweise derart ausgebildet, dass die akustische Kopplung zwischen dem integrierten Ultraschallwandler und dem weiteren Ultraschallwandler gewährleistet ist.

Das erfindungsgemäße Verfahren für ein Bereitstellen eines röntgenographischen Bilddatensatzes und eines sonographischen Bilddatensatzes einer Brust einer Patientin mittels einer Mammographieanlage umfasst folgende Schritte:
- Positionieren der Brust der Patientin in einem Brustaufnahmebereich der Mammographieanlage,
- Erfassen des röntgenographischen Bilddatensatzes der Brust der Patientin mittels eines Röntgenstrahlers und eines integrierten Röntgendetektors der Mammographieanlage,
- Erfassen des sonographischen Bilddatensatzes der Brust der Patientin mittels eines flächigen, integrierten Ultraschallwandlers einer Stützvorrichtung der Mammographieanlage, wobei zwischen dem Erfassen der Bilddatensätze die Stützvorrichtung die Brust der Patientin fixiert hält, und
- Bereitstellen des röntgenographischen Bilddatensatzes und des sonographischen Bilddatensatzes.

Die Brust der Patientin wird vorzugsweise in einer stehenden Pose derart in dem Brustaufnahmebereich positioniert, dass der integrierte Ultraschallwandler vorzugsweise die Brust flächig, insbesondere in einem größeren Bereich kontaktiert, beispielsweise durch die Kompression mittels der ersten Kompressionsvorrichtung und/oder der zweiten Kompressionsvorrichtung. Grundsätzlich ist auch denkbar, dass die Mammographieanlage für die Patientin in liegender Pose entsprechend ausgebildet ist.

Der röntgenographische Bilddatensatz wird vorzugsweise gemäß der röntgenographischen Bildgebung und der sonographische Bilddatensatz gemäß der sonographischen Bildgebung erfasst und entsprechende röntgenographische Bilder sowie sonographische Bilder rekonstruiert.

Zwischen dem Erfassen der Bilddatensätze hält die Stützvorrichtung die Brust der Patientin vorzugsweise derart fixiert, dass die jeweiligen Bilddatensätze in unmittelbarer Abfolge nacheinander, in einer beliebigen Reihenfolge erfasst werden können, wobei entweder zunächst der röntgenographische Bilddatensatz und danach der sonographische Bilddatensatz oder zunächst der sonographische Bilddatensatz und danach der röntgenographische Bilddatensatz erfasst werden. Das Fixieren der Brust der Patientin bedeutet insbesondere, dass die Brust der Patientin zwischen dem Erfassen der Bilddatensätze nicht repositioniert oder umgelagert werden muss. Eine Pause zwischen dem Erfassen der Bilddatensätze ist vorzugsweise vergleichsweise kurz, sprich der eine Bilddatensatz wird vorzugsweise unmittelbar nach dem anderen Bilddatensatz aufgenommen. Grundsätzlich ist es denkbar, dass die Bilddatensätze ohne Pause, sprich in Abhängigkeit von einer jeweiligen Messdauer quasi gleichzeitig, erfasst werden.

Eine Ausführungsform sieht vor, dass das Erfassen des sonographischen Bilddatensatzes ein Ermitteln einer Deformation des flächigen, integrierten Ultraschallwandlers umfasst und dass das Ermitteln der Deformation des flächigen, integrierten Ultraschallwandlers folgende Schritte umfasst:
- Ansteuern zumindest eines Ultraschallwandlerelements des flächigen, integrierten Ultraschallwandlers derart, dass Ultraschallwellen ausgesendet werden,
- Erfassen einer Laufzeit der Ultraschallwellen mittels des zumindest einen Ultraschallwandlerelements und
- Ermitteln der Deformation des flächigen, integrierten Ultraschallwandlers gemäß der Laufzeit der Ultraschallwellen. Das Ansteuern des zumindest einen Ultraschallwandlerelements kann einzeln oder gruppenweise erfolgen. Grundsätzlich können auch alle Ultraschallwandlerelemente gleichzeitig oder in einer vorgegebenen Reihenfolge angesteuert werden. Vorzugsweise sind die jeweiligen Ultraschallwandlerelemente zum Erfassen der Ultraschallwellen ausgebildet und beispielsweise ist die sonographische Steuereinheit zu einem Berechnen der Laufzeit ausgebildet. Das Ermitteln der Deformation kann insbesondere eine Information über einen Abstand zwischen den Ultraschallwandlerelementen, vorzugsweise im starren bzw. planaren Zustand des integrierten Ultraschallwandlers, zueinander und/oder über einen Abstand zu einem Referenzobjekt und/oder eine Randbedingung, dass an dem Referenzobjekt eine komplette Reflexion der Ultraschallwellen erfolgt, bedingen. Das Referenzobjekt kann beispielsweise die erste Kompressionsvorrichtung, die zweite Kompressionsvorrichtung und/oder der integrierten Röntgendetektor sein, wobei deren Geometrie beispielsweise durch Maßzeichnungen oder Bildaufnahmen erfasst ist. Beispielsweise kann das Ermitteln der Deformation mittels eines iterativen Algorithmus erfolgen, wobei der Algorithmus derart angepasst wird, dass das in dem sonographischen Bilddatensatz dargestellte Referenzobjekt vorzugsweise der erfassten Geometrie entspricht. Vorzugsweise kann gemäß der Anpassung des Algorithmus, insbesondere der Eingangsparameter des Algorithmus, die Deformation des integrierten Ultraschallwandlers ermittelt werden. Das Ermitteln der Deformation kann grundsätzlich während der sonographischen Bildgebung oder bei einer weiteren sonographischen Bildgebung für eine Kalibrierung der Mammographieanlage erfolgen.

Eine Ausführungsform sieht vor, dass das Ermitteln der Deformation des flächigen, integrierten Ultraschallwandlers unter Verwendung des erfassten röntgenographischen Bilddatensatzes erfolgt. Insbesondere kann die sonographische Steuereinheit und/oder die Steuereinheit der Mammographieanlage zu einem Ermitteln der Deformation des integrierten Ultraschallwandlers mittels eines zweidimensionalen oder dreidimensionalen röntgenographischen Bilddatensatzes ausgebildet sein. Weil typischerweise der integrierte Ultraschallwandler nicht zu 100% röntgentransparent ist, kann die Deformation des integrierten Ultraschallwandlers aus dem röntgenographischen Bilddatensatz ermittelt und für die sonographische Bildgebung verwendet werden. Alternativ oder zusätzlich kann eine Hautlinie, sprich die Oberfläche, der Brust der Patientin für das Ermitteln der Deformation berücksichtigt werden. Typischerweise kann die Deformation des integrierten Ultraschallwandlers mittels der zweidimensionalen oder dreidimensionalen röntgenographischen Bildgebung, insbesondere mittels der röntgenographischen Tomosynthese, ermittelt werden. Alternativ oder zusätzlich kann der integrierte Ultraschallwandler über einige röntgenstrahlenabsorbierende Markierungen verfügen, welche in der röntgenographischen Bildgebung sichtbar sind. In diesem Fall ist vorzugsweise ein planarer Abstand X zwischen den röntgenbasierenden Markierungen im starren bzw. planaren Zustand des integrierten Ultraschallwandlers bekannt. Unter Verwendung des röntgenographischen Bilddatensatzes kann vorzugsweise ein deformierter Abstand X' erfasst werden, wobei die Deformation des integrierten Ultraschallwandlers unter Verwendung des planaren Abstands X und des deformierten Abstands X' ermittelt wird. Grundsätzlich ist es denkbar, dass die Deformation des flächigen, integrierten Ultraschallwandlers unter Verwendung des erfassten röntgenographischen Bilddatensatzes und mittels des Erfassens der Laufzeit der Ultraschallwellen in Kombination erfolgt.

Vorzugsweise wird durch das Ermitteln der Deformation des integrierten Ultraschallwandlers die sonographische Bildgebung ermöglicht, besonders bevorzugt vergleichsweise verbessert. Ein weiterer Vorteil kann sein, dass gemäß der ermittelten Deformation des integrierten Ultraschallwandlers eine dreidimensionale sonographische Bildgebung verbessert werden kann. Alternativ oder zusätzlich können gemäß der Deformation des integrierten Ultraschallwandlers die Ultraschallwellen auf einen Bereich fokussiert werden, wodurch neben der sonographischen Bildgebung und der röntgenographischen Bildgebung eine sonographische Therapie mittels der Mammographieanlage möglich sein kann.

Eine Ausführungsform sieht vor, dass der sonographische Bilddatensatz gemäß der Deformation des flächigen, integrierten Ultraschallwandlers rekonstruiert wird. Vorzugsweise kann die Steuereinheit der Mammographieanlage derart ausgebildet sein, dass der sonographische Bilddatensatz gemäß der Deformation des integrierten Ultraschallwandlers rekonstruiert werden kann. Die Steuereinheit der Mammographieanlage kann derart in Programmcodemitteln abgebildet sein, dass die Recheneinheit der Mammographieanlage die Programmcodemittel zum Rekonstruieren des sonographischen Bilddatensatz und/oder des röntgenographischen Bilddatensatzes ausführen kann. Vorzugsweise kann auf einem Bildschirm der Mammographieanlage der sonographische Bilddatensatz und/oder der röntgenographische Bilddatensatz für eine Befundung oder Diagnose bereitgestellt bzw. angezeigt werden. Alternativ oder zusätzlich kann der sonographische Bilddatensatz und/oder der röntgenographische Bilddatensatz beispielsweise auf einem Server gespeichert und wieder abgerufen werden.

Eine Ausführungsform sieht vor, dass eine Deformation der Stützvorrichtung mittels des flächigen, integrierten Ultraschallwandlers erfasst wird und dass der röntgenographische Bilddatensatz gemäß der Deformation der Stützvorrichtung rekonstruiert wird. Ein Vorteil besteht darin, dass mittels des integrierten Ultraschallwandlers ein Kissen, insbesondere ein Ultraschallgelkissen, erfasst werden kann und mittels des erfassten Kissens der röntgenographische Bilddatensatz angepasst bzw. korrigiert werden kann. Dies ist insbesondere im Hinblick auf eine Korrektur der Absorption der Röntgenstrahlen in Bezug auf das Kissen vorteilhaft.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert.

Es zeigen:
Fig. 1 eine Mammographieanlage in einem ersten Ausführungsbeispiel,
Fig. 2 eine Mammographieanlage in einem zweiten Ausführungsbeispiel,
Fig. 3 eine Mammographieanlage in einem dritten Ausführungsbeispiel,
Fig. 4 eine Mammographieanlage in einem vierten Ausführungsbeispiel,
Fig. 5 eine Mammographieanlage in einem fünften Ausführungsbeispiel,
Fig. 6 eine Mammographieanlage in einem sechsten Ausführungsbeispiel,
Fig. 7 eine schematische Ansicht des integrierten Ultraschallwandlers 16 in Vogelperspektive,
Fig. 8 ein erstes Flussdiagramm des Verfahrens und
Fig. 9 ein zweites Flussdiagramm des Verfahrens.

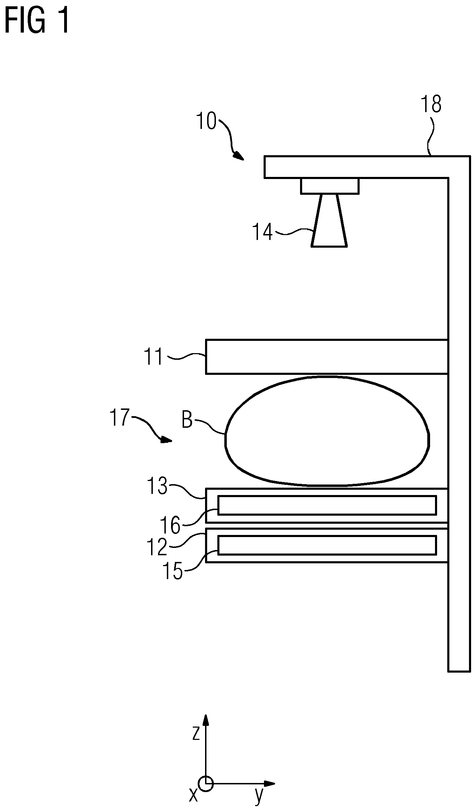

**Fig. 1** zeigt eine Mammographieanlage 10 zur röntgenographischen und sonographischen Bildgebung einer Brust B einer Patientin in einer Seitenansicht der Mammographieanlage 10. Die Mammographieanlage 10 weist eine erste Kompressionsvorrichtung 11, eine zweite Kompressionsvorrichtung 12, eine Stützvorrichtung 13, einen Röntgenstrahler 14, einen integrierten Röntgendetektor 15 und einen integrierten Ultraschallwandler 16 auf, welcher flächig ausgebildet ist. Die zweite Kompressionsvorrichtung 12 weist den integrierten Röntgendetektor 15 auf. Die Stützvorrichtung 13 ist in diesem Ausführungsbeispiel an der zweiten Kompressionsvorrichtung 12 angeordnet. Zwischen der Stützvorrichtung 13 und der ersten Kompressionsvorrichtung 11 ist ein Brustaufnahmebereich 17 vorgesehen. Die Brust B der Patientin ist in diesem Fall im Brustaufnahmebereich 17 positioniert. Die Stützvorrichtung 13 weist den integrierten Ultraschallwandler 16 auf und
der integrierte Ultraschallwandler 16 ist in Richtung des Brustaufnahmebereichs 17 ausgerichtet. Grundsätzlich ist es denkbar, dass die Stützvorrichtung 13 fest in eine der beiden Kompressionsvorrichtung fest integriert ist, in diesem Fall also in die zweite Kompressionsvorrichtung 12. Die erste Kompressionsvorrichtung 11 und die zweite Kompressionsvorrichtung 12 sind an einem Stativ 18 der Mammographieanlage 10 höhenverstellbar und für eine Kompression der Brust B angeordnet. Die Brust B der Patienten ist lediglich schematisch dargestellt. Bezüglich des in Fig. 1 gezeigten Koordinatensystems ist in diesem Ausführungsbeispiel eine Schulter der Patientin parallel zur x-Achse ausgerichtet und eine Achse zwischen Pectoralis und Mamille entlang der y-Achse. Die Körperachse der Patienten ist parallel zur z-Achse. Der integrierte Ultraschallwandler 16 ist bezogen auf die x-y-Ebene flächig ausgebildet. In diesem Ausführungsbeispiel ist der integrierte Ultraschallwandler 16 starr bzw. planar ausgebildet. Die Systemachse der Mammographieanlage 10 entspricht der z-Achse.

Grundsätzlich werden in der folgenden Figurenbeschreibung im Wesentlichen gleich bleibende Strukturen und Einheiten mit den in Fig. 1 ursprünglich gezeigten Bezugszeichen benannt.

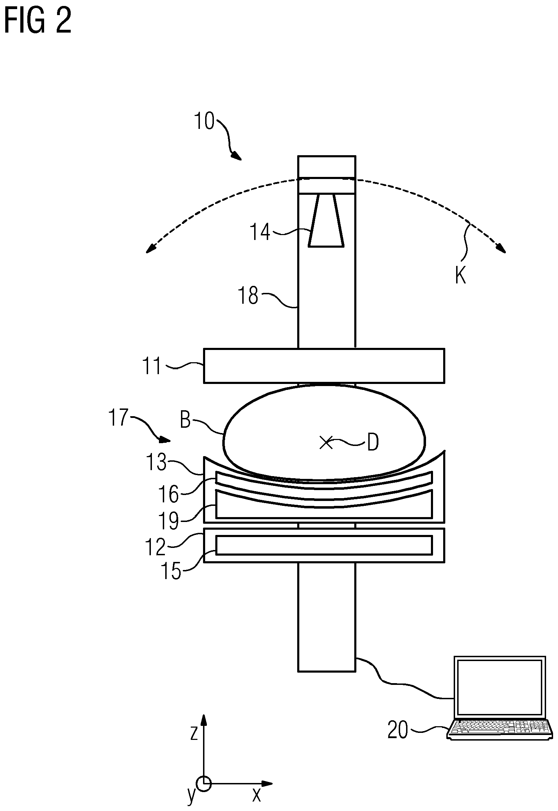

**Fig. 2** zeigt die Mammographieanlage 10 in einer Frontalansicht in einem zweiten Ausführungsbeispiel, wobei die Stützvorrichtung 13 und der integrierte Ultraschallwandler 16 derart deformierbar ausgebildet sind, dass sie an die Brust B der Patientin anformbar sind. In diesem Ausführungsbeispiel ist die Stützvorrichtung 13 und der integrierte Ultraschallwandler 16 an die Brust B angeformt, wodurch eine akustische Kopplung im Vergleich zum Ausführungsbeispiel in Fig. 1 erhöht ist.

Die Mammographieanlage 10 weist eine Recheneinheit 20 auf, wobei die Mammographieanlage 10 zu einem Ermitteln einer Deformation des integrierten Ultraschallwandlers 16 ausgebildet ist und die Recheneinheit 20 zu einem Rekonstruieren eines sonographischen Bilddatensatzes gemäß der Deformation ausgebildet ist. Die Recheneinheit 20 weist einen Monitor für eine Anzeige des röntgenographischen Bilddatensatzes und des sonographischen Bilddatensatzes auf.

Die Stützvorrichtung 13 weist ein Kissen 19 auf. Die Stützvorrichtung 13 weist eine dem Brustaufnahmebereich 17 zugewandte erste Seite und eine derjenigen Kompressionsvorrichtung 12, an der die Stützvorrichtung 13 angeordnet ist, zugewandte zweite Seite aufweist, wobei die erste Seite den integrierten Ultraschallwandler 16 aufweist. Die zweite Seite weist das Kissen 19 auf. Das Kissen 19 ist also zwischen der zweiten Kompressionsvorrichtung 12 und dem integrierten Ultraschallwandler 16 angeordnet.

Der Röntgenstrahler 14 ist auf einem Kreisbogen K verschiebbar angeordnet, wobei der Kreisbogen K um eine Drehachse D definiert ist, welche senkrecht zur Systemachse der Mammographieanlage 10 ausgerichtet ist. In diesem Fall ist die Drehachse D parallel zur y-Achse. Der Röntgenstrahler 14 kann einen Winkelbereich entlang des Kreisbogens K mit einer Länge von bis zu 90° abfahren.

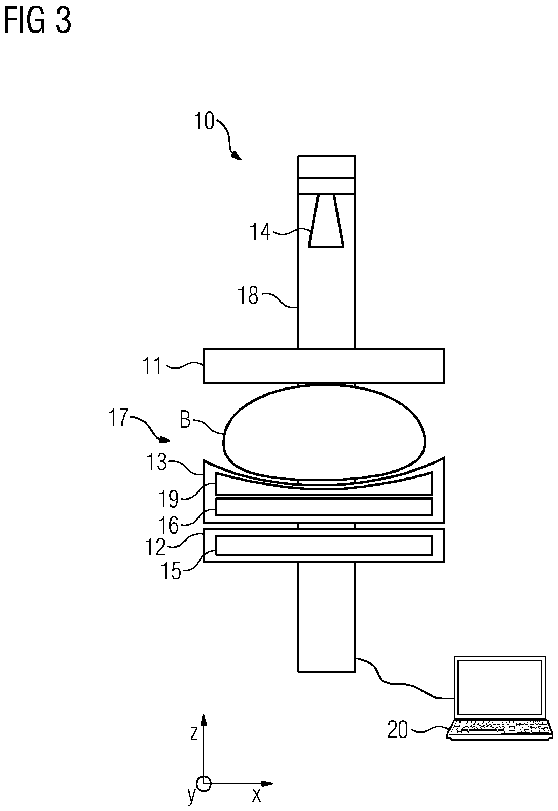

Im Vergleich zur Fig. 1 ist die Mammographieanlage 10 in Fig. 2 und in Fig. 3 um 90° um die Systemachse gedreht dargestellt. Fig. 2 und Fig. 3 zeigen außerdem, dass die Stützvorrichtung 13, insbesondere der integrierte Ultraschallwandler 16, entlang aller Raumachsen deformierbar ist.

**Fig. 3** zeigt die Mammographieanlage 10 in einer Frontalansicht in einem dritten Ausführungsbeispiel und eine im Vergleich zur Fig. 2 alternative Ausführung. In diesem Ausführungsbeispiel weist die zweite Seite der Stützvorrichtung 13 den integrierten Ultraschallwandler 16 auf. Die erste Seite der Stützvorrichtung 13 weist das Kissen 19 auf. Das Kissen 19 ist also zwischen dem Brustaufnahmebereich 17 und dem integrierten Ultraschallwandler 16 angeordnet. Die Stützvorrichtung 13 ist typischerweise durch das Kissen 19 an die Brust B anformbar. Das Kissen 19 ist vorzugsweise als Ultraschallgelkissen ausgebildet oder weist vergleichbare akustische Eigenschaften wie ein Ultraschallgelkissen auf. Dies ist insbesondere im Hinblick auf die akustische Kopplung vorteilhaft.

In diesem Ausführungsbeispiel ist die Stützvorrichtung 13 an die Brust B angeformt, wodurch die akustische Kopplung im Vergleich zum Ausführungsbeispiel in Fig. 1 erhöht ist.

Die Mammographieanlage 10 weist die Recheneinheit 20 auf, wobei der integrierte Ultraschallwandler 16 zu einem Erfassen einer Deformation der Stützvorrichtung 13 ausgebildet ist und die Recheneinheit 20 zu einem Rekonstruieren eines röntgenographischen Bilddatensatzes gemäß der Deformation der Stützvorrichtung 13 ausgebildet ist. Der röntgenographische Bilddatensatz und der sonographische Bilddatensatz werden auf dem Monitor der Recheneinheit 20 angezeigt.

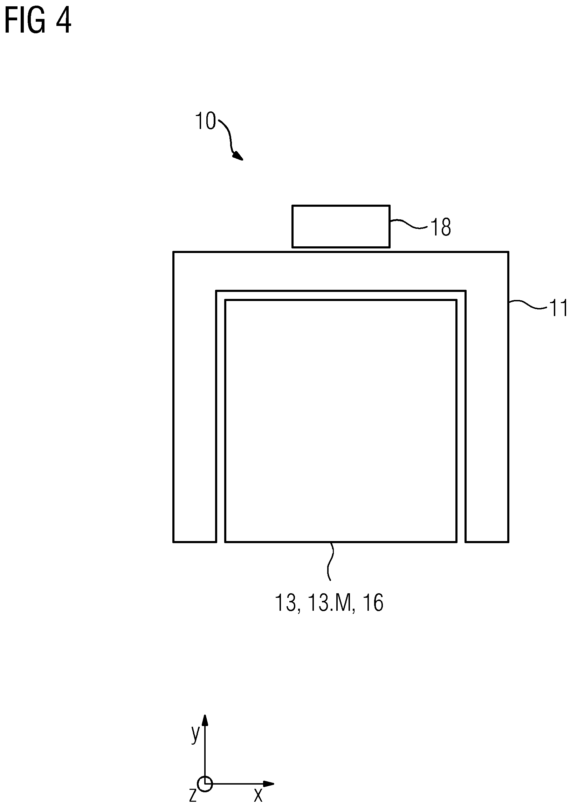

**Fig. 4** zeigt die Mammographieanlage 10 in einer Ansicht aus der Vogelperspektive in einem vierten Ausführungsbeispiel. In diesem Ausführungsbeispiel ist Stützvorrichtung 13 an der ersten Kompressionsvorrichtung 11 angeordnet. Die Stützvorrichtung 13 weist eine Membran 13.M auf, wobei an der Membran 13.M der integrierte Ultraschallwandler 16 angeordnet ist.

Die stützende Kompressionsvorrichtung, in diesem Ausführungsbeispiel also die erste Kompressionsvorrichtung 11, ist als ein Rahmen ausgebildet. In diesem Fall ist der Rahmen auf einer Seite offen, sprich die stützende Kompressionsvorrichtung ist c-förmig bzw. u-förmig ausgebildet. Die Stützvorrichtung 13 und damit der integrierte Ultraschallwandler 16 sind innerhalb des Rahmens eingespannt bzw. angeordnet.

Fig. 4 zeigt schematisch die flächige Ausdehnung des integrierten Ultraschallwandlers 16 in der x-y-Ebene.

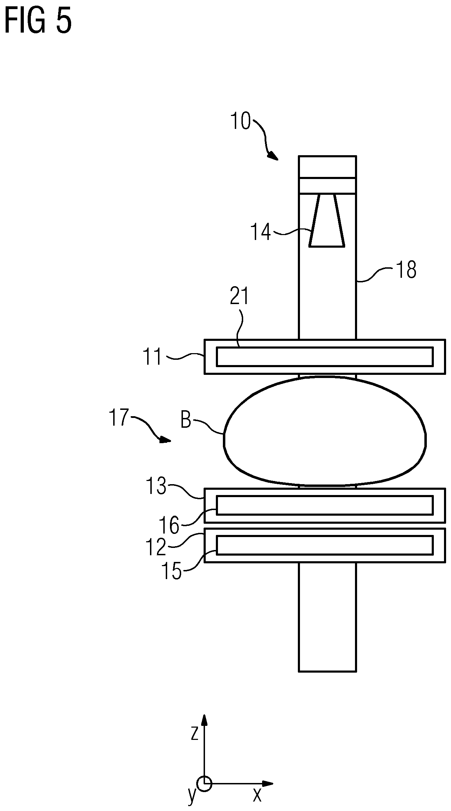

**Fig. 5** zeigt, dass die Mammographieanlage 10 zusätzlich zu dem integrierten Ultraschallwandler 16 einen weiteren Ultraschwallwandler 21 aufweist, wobei der weitere Ultraschallwandler 21 derart angeordnet ist, dass der Brustaufnahmebereich 17 zwischen dem integrierten Ultraschallwandler 16 und dem weiteren Ultraschallwandler 21 vorgesehen ist. Vorzugsweise kann einer der beiden Ultraschallwandler jeweils Ultraschwallwellen senden und/oder empfangen.

In diesem Ausführungsbeispiel ist der weitere Ultraschallwandler 21 in die erste Kompressionsvorrichtung 11 integriert. Alternativ könnte eine weitere Stützvorrichtung den weiteren Ultraschallwandler 21 aufweisen, wobei die weitere Stützvorrichtung an der ersten Kompressionsvorrichtung 11 angeordnet ist. Die obigen Ausführungen in Bezug auf den Ultraschallwandler 16 treffen im Wesentlichen, insbesondere die Anordnung innerhalb einer als Rahmen ausgebildeten Kompressionsvorrichtung, auch auf den weiteren Ultraschallwandler 21 zu.

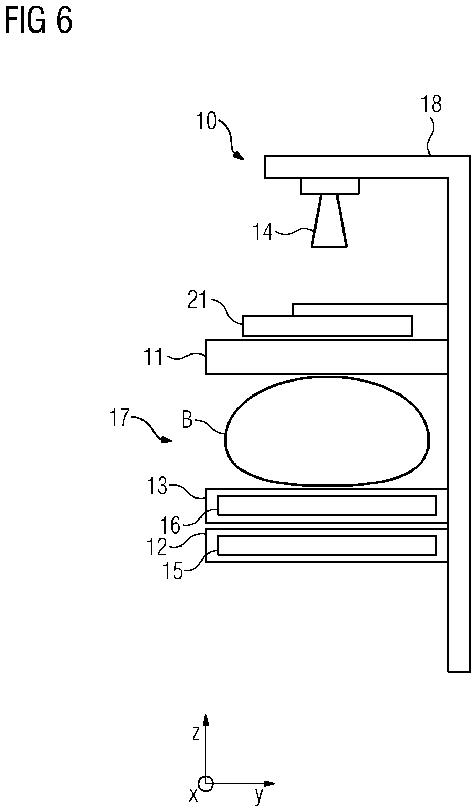

**Fig. 6** zeigt eine im Vergleich zur Fig. 5 alternative Ausführung. Der weitere Ultraschallwandler 21 ist zwischen dem Röntgenstrahler 14 und der ersten Kompressionsvorrichtung 11 angeordnet und mittels eines weiteren Stativs mit dem Stativ 18 verbunden. Grundsätzlich ist es denkbar, dass der weitere Ultraschallwandler 21, während der röntgenographischen Bildgebung außerhalb eines Strahlengangs angeordnet ist, welcher durch den Röntgenstrahler 14 und den integrierten Röntgendetektor 15 vorgegeben ist, und für die sonographische Bildgebung in den Strahlengang verfahren wird.

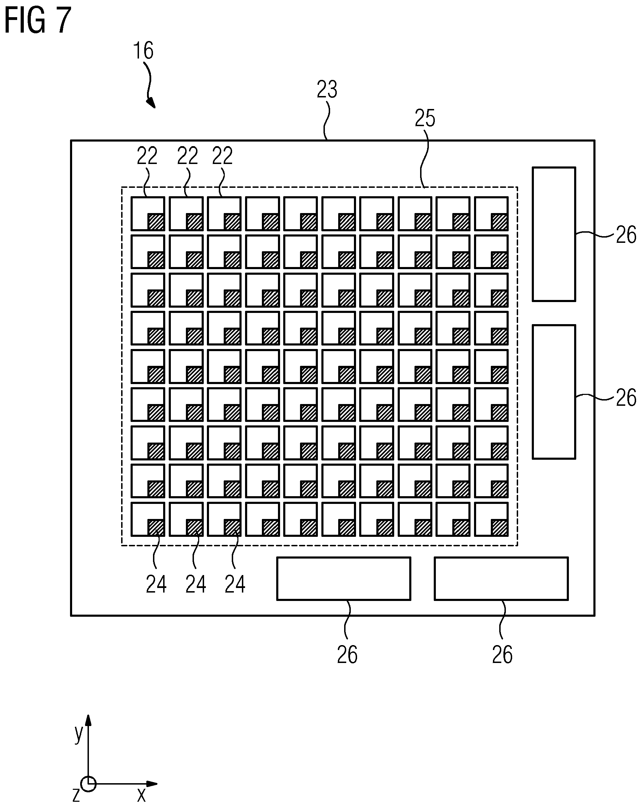

**Fig. 7** zeigt eine schematische Ansicht des integrierten Ultraschallwandlers 16 in Vogelperspektive. Der integrierte Ultraschallwandler 16 basiert auf der pMUT-Technologie. Die Ultraschallwandlerelemente 22 sind matrixförmig in der x-y-Ebene auf einem Substrat 23 angeordnet und gleichmäßig zueinander beabstandet. Das Substrat 23 kann Textil, Glas, Folie, Gewebe und/oder Gaze aufweisen. In einem Fall kann das Substrat 23 der Membran 13.M der Stützvorrichtung 13 entsprechen, in einem anderen Fall ist das Substrat 23 zusätzlich zu der Membran 13.M an der Stützvorrichtung 13 angeordnet. Jedes Ultraschallwandlerelement 22 entspricht einem Pixel und weist ein Steuerelement 24 zum Ansteuern des jeweiligen Pixels auf. Jedes Steuerelement 24 weist vorzugsweise einen Transistor auf. Das Substrat 23 weist einen belichteten Bereich 25 auf, welcher die Ultraschallwandlerelemente 22 umfasst. Eine Ultraschallwandlersteuereinheit 26, welche außerhalb des belichteten Bereichs 25 angeordnet sind, ist zu einem Ansteuern der Ultraschallwandlerelemente 22 ausgebildet. Das Ansteuern kann einzeln oder gruppenweise erfolgen. Der Strahlengang des Röntgenstrahlers 14 beleuchtet vorzugsweise ausschließlich den belichteten Bereich 25. In diesem Fall ist die Ultraschallwandlersteuereinheit 26, welche typischerweise elektronische und damit potentiell auf Röntgenstrahlen sensitive Bauteile aufweist, von den Röntgenstrahlen ausgenommen bzw. nicht durchleuchtet.

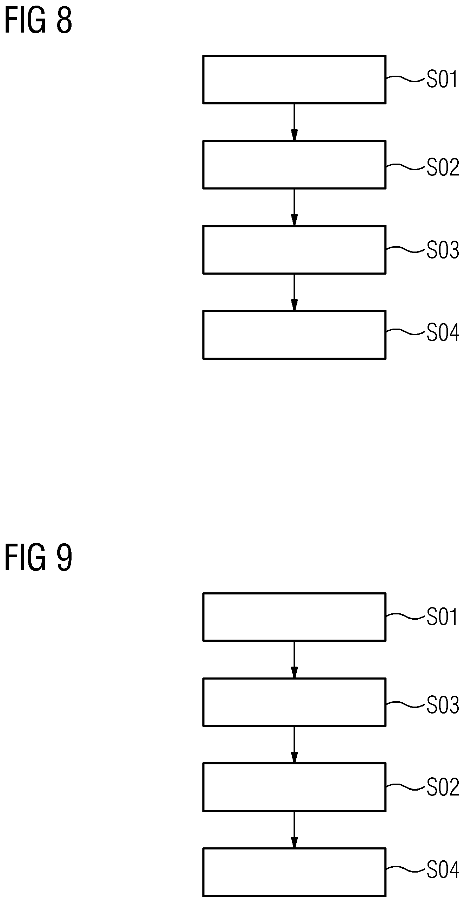

**Fig. 8** zeigt ein erstes Flussdiagramm des Verfahrens für ein Bereitstellen eines röntgenographischen Bilddatensatzes und eines sonographischen Bilddatensatzes einer Brust B einer Patientin mittels einer Mammographieanlage 10.

In diesem Ausführungsbeispiel wird der röntgenographische Bilddatensatz vor dem sonographischen Bilddatensatz erfasst.

Verfahrensschritt S01 kennzeichnet das Positionieren der Brust B der Patientin in einem Brustaufnahmebereich 17 der Mammographieanlage 10.

Verfahrensschritt S02 kennzeichnet das Erfassen des röntgenographischen Bilddatensatzes der Brust B der Patientin mittels eines Röntgenstrahlers 14 und eines integrierten Röntgendetektors 16 der Mammographieanlage 10.

Verfahrensschritt S03 kennzeichnet das Erfassen des sonographischen Bilddatensatzes der Brust B der Patientin mittels eines flächigen, integrierten Ultraschallwandlers 16 einer Stützvorrichtung 13 der Mammographieanlage 10, wobei zwischen dem Erfassen der Bilddatensätze die Stützvorrichtung 13 die Brust B der Patientin fixiert hält.

Verfahrensschritt S04 kennzeichnet das Bereitstellen des röntgenographischen Bilddatensatzes und des sonographischen Bilddatensatzes.

**Fig. 9** zeigt ein zweites Flussdiagramm des Verfahrens für ein Bereitstellen eines röntgenographischen Bilddatensatzes und eines sonographischen Bilddatensatzes einer Brust B einer Patientin mittels einer Mammographieanlage 10.

In diesem Ausführungsbeispiel wird im Vergleich zum Ausführungsbeispiel in Fig. 8 der sonographische Bilddatensatz vor dem röntgenographischen Bilddatensatz erfasst. Grundsätzlich ist es denkbar, dass der sonographische Bilddatensatz und der röntgenographische Bilddatensatz quasi gleichzeitig erfasst werden.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Mammographieanlage (10) zur röntgenographischen und sonographischen Bildgebung einer Brust (B) einer Patientin, aufweisend
- eine erste Kompressionsvorrichtung (11),
- eine zweite Kompressionsvorrichtung (12),
- eine Stützvorrichtung (13),
- einen Röntgenstrahler (14),
- einen integrierten Röntgendetektor (15) und
- einen integrierten Ultraschallwandler (16), welcher flächig ausgebildet ist,
- wobei die zweite Kompressionsvorrichtung (12) den integrierten Röntgendetektor (16) aufweist,
- wobei die Stützvorrichtung (13) an der ersten Kompressionsvorrichtung (11) oder an der zweiten Kompressionsvorrichtung (12) angeordnet ist und
- wobei zwischen der Stützvorrichtung (13) und derjenigen Kompressionsvorrichtung (11, 12), die der Kompressionsvorrichtung (12, 11), an welcher die Stützvorrichtung (13) angeordnet ist, gegenüberliegt, ein Brustaufnahmebereich (17) vorgesehen ist und die Brust (B) der Patientin im Brustaufnahmebereich (17) positionierbar ist,
- wobei die Stützvorrichtung (13) den integrierten Ultraschallwandler (16) aufweist und
- wobei der integrierte Ultraschallwandler (16) in Richtung des Brustaufnahmebereichs (17) ausgerichtet ist,
**dadurch gekennzeichnet,**
**dass** die Stützvorrichtung (13) und der integrierte Ultraschallwandler (16) derart deformierbar ausgebildet sind, dass sie an die Brust (B) der Patientin anformbar sind, wobei der integrierte Ultraschallwandler (16) auf einem elastischen Substrat angeordnet ist.

2. Mammographieanlage (10) nach Anspruch 1, **dadurch gekennzeichnet, dass** die Mammographieanlage (10) eine Recheneinheit (20) aufweist, wobei die Mammographieanlage (10) zu einem Ermitteln einer Deformation des integrierten Ultraschallwandlers (16) ausgebildet ist und die Recheneinheit (20) zu einem Rekonstruieren eines sonographischen Bilddatensatzes gemäß der Deformation ausgebildet ist.

3. Mammographieanlage (10) nach einem der vorhergehenden Ansprüche, **dadurch gekennzeichnet, dass** die Stützvorrichtung (13) eine Membran aufweist, wobei an der Membran der integrierte Ultraschallwandler angeordnet ist.

4. Mammographieanlage (10) nach einem der vorhergehenden Ansprüche, **dadurch gekennzeichnet, dass** diejenige Kompressionsvorrichtung (11, 12), an welcher die Stützvorrichtung (13) angeordnet ist, als ein Rahmen ausgebildet ist und die Stützvorrichtung (13) innerhalb des Rahmens eingespannt ist.

5. Mammographieanlage (10) nach einem der vorhergehenden Ansprüche, **dadurch gekennzeichnet, dass** die Stützvorrichtung (13) ein Kissen (19) aufweist.

6. Mammographieanlage (10) nach einem der vorhergehenden Ansprüche, **dadurch gekennzeichnet, dass** die Stützvorrichtung (13) eine dem Brustaufnahmebereich (17) zugewandte erste Seite und eine derjenigen Kompressionsvorrichtung (11, 12), an der die Stützvorrichtung (13) angeordnet ist, zugewandte zweite Seite aufweist, wobei die erste Seite den integrierten Ultraschallwandler (16) aufweist.

7. Mammographieanlage (10) nach den Ansprüchen 5 und 6, **dadurch gekennzeichnet, dass** die zweite Seite der Stützvorrichtung (13) das Kissen (19) aufweist.

8. Mammographieanlage (10) nach einem der Ansprüche 1 bis 5, **dadurch gekennzeichnet, dass** die Stützvorrichtung (13) eine dem Brustaufnahmebereich (17) zugewandte erste Seite und eine derjenigen Kompressionsvorrichtung (11, 12), an der die Stützvorrichtung (13) angeordnet ist, zugewandte zweite Seite aufweist, wobei die zweite Seite den integrierten Ultraschallwandler (16) aufweist.

9. Mammographieanlage (10) nach den Ansprüchen 5 und 8, **dadurch gekennzeichnet, dass** die erste Seite der Stützvorrichtung (13) das Kissen (19) aufweist.

10. Mammographieanlage (10) nach einem der vorhergehenden Ansprüche, **dadurch gekennzeichnet, dass** die Mammographieanlage (10) eine Recheneinheit (20) aufweist, wobei der integrierte Ultraschallwandler (16) zu einem Erfassen einer Deformation der Stützvorrichtung (13) ausgebildet ist und die Recheneinheit (20) zu einem Rekonstruieren eines röntgenographischen Bilddatensatzes gemäß der Deformation der Stützvorrichtung (13) ausgebildet ist.

11. Mammographieanlage (10) nach einem der vorhergehenden Ansprüche, **dadurch gekennzeichnet, dass** die Mammographieanlage (10) zusätzlich zu dem integrierten Ultraschallwandler (16) einen weiteren Ultraschwallwandler (21) aufweist, wobei der weitere Ultraschallwandler (21) derart angeordnet ist, dass der Brustaufnahmebereich (17) zwischen dem integrierten Ultraschallwandler (16) und dem weiteren Ultraschallwandler (21) vorgesehen ist.

12. Verfahren für ein Bereitstellen eines röntgenographischen Bilddatensatzes und eines sonographischen Bilddatensatzes einer Brust (B) einer Patientin mittels einer Mammographieanlage (10), mit den folgenden Schritten:
- Positionieren der Brust (10) der Patientin in einem Brustaufnahmebereich (17) der Mammographieanlage (10),
- Erfassen des röntgenographischen Bilddatensatzes der Brust (B) der Patientin mittels eines Röntgenstrahlers (14) und eines integrierten Röntgendetektors (15) der Mammographieanlage (10) ,
- Erfassen des sonographischen Bilddatensatzes der Brust (B) der Patientin mittels eines flächigen, integrierten Ultraschallwandlers (16) einer Stützvorrichtung (13) der Mammographieanlage (10), wobei zwischen dem Erfassen der Bilddatensätze die Stützvorrichtung (13) die Brust (B) der Patientin fixiert hält, und
- Bereitstellen des röntgenographischen Bilddatensatzes und des sonographischen Bilddatensatzes,
**dadurch gekennzeichnet,**
**dass** die Stützvorrichtung (13) und der flächige, integrierte Ultraschallwandler (16) derart deformierbar ausgebildet sind, dass sie an die Brust (B) der Patientin anformbar sind, wobei der flächige, integrierte Ultraschallwandler (16) auf einem elastischen Substrat angeordnet ist.

13. Verfahren nach Anspruch 12, wobei das Erfassen des sonographischen Bilddatensatzes ein Ermitteln einer Deformation des flächigen, integrierten Ultraschallwandlers (16) umfasst und wobei das Ermitteln der Deformation des flächigen, integrierten Ultraschallwandlers (16) folgende Schritte umfasst:
- Ansteuern zumindest eines Ultraschallwandlerelements (22) des flächigen, integrierten Ultraschallwandlers (16) derart, dass Ultraschallwellen ausgesendet werden,
- Erfassen einer Laufzeit der Ultraschallwellen mittels des zumindest einen Ultraschallwandlerelements (22) und
- Ermitteln der Deformation des flächigen, integrierten Ultraschallwandlers (16) gemäß der Laufzeit der Ultraschallwellen.

14. Verfahren nach einem der Ansprüche 12 bis 13, wobei das Ermitteln der Deformation des flächigen, integrierten Ultraschallwandlers (16) unter Verwendung des erfassten röntgenographischen Bilddatensatzes erfolgt.

15. Verfahren nach einem der Ansprüche 12 bis 14, wobei eine Deformation der Stützvorrichtung (13) mittels des flächigen, integrierten Ultraschallwandlers (16) erfasst wird und wobei der röntgenographische Bilddatensatz gemäß der Deformation der Stützvorrichtung (13) rekonstruiert wird.

## Claims

1. Mammography installation (10) for radiographic and ultrasonic imaging of a breast (B) of a patient, comprising
- a first compression device (11),
- a second compression device (12),
- a support device (13),
- an X-ray emitter (14),
- an integrated X-ray detector (15) and
- an integrated ultrasound transducer (16) which is designed as an even layer,
- wherein the second compression device (12) comprises the integrated X-ray detector (16),
- wherein the support device (13) is arranged at the first compression device (11) or at the second compression device (12) and
- wherein a breast locating region (17) is provided between the support device (13) and the compression device (11, 12) that is situated opposite the compression device (12, 11) at which the support device (13) is arranged, and the breast (B) of the patient can be positioned in the breast locating region (17),
- wherein the support device (13) comprises the integrated ultrasound transducer (16), and
- wherein the integrated ultrasound transducer (16) is oriented in the direction of the breast locating region (17),
**characterised in that**
the support device (13) and the integrated ultrasound transducer (16) are designed to be deformable such that they can be moulded to the breast (B) of the patient, wherein the integrated ultrasound transducer (16) is arranged on an elastic substrate.

2. Mammography installation (10) according to claim 1, **characterised in that** the mammography installation (10) has a computing unit (20), wherein the mammography installation (10) is designed to determine a deformation of the integrated ultrasound transducer (16) and the computing unit (20) is designed to reconstruct an ultrasonic image data record in accordance with the deformation.

3. Mammography installation (10) according to one of the preceding claims, **characterised in that** the support device (13) has a membrane, wherein the integrated ultrasound transducer is arranged at the membrane.

4. Mammography installation (10) according to one of the preceding claims, **characterised in that** the compression device (11, 12) at which the support device (13) is arranged is designed as a frame and the support device (13) is mounted within the frame.

5. Mammography installation (10) according to one of the preceding claims, **characterised in that** the support device (13) comprises a cushion (19).

6. Mammography installation (10) according to one of the preceding claims, **characterised in that** the support device (13) has a first side facing the breast locating region (17) and a second side facing the compression device (11, 12) at which the support device (13) is arranged, wherein the first side comprises the integrated ultrasound transducer (16).

7. Mammography installation (10) according to claims 5 and 6, **characterised in that** the second side of the support device (13) comprises the cushion (19).

8. Mammography installation (10) according to one of the claims 1 to 5, **characterised in that** the support device (13) has a first side facing the breast locating region (17) and a second side facing the compression device (11, 12) at which the support device (13) is arranged, wherein the second side comprises the integrated ultrasound transducer (16).

9. Mammography installation (10) according to claims 5 and 8, **characterised in that** the first side of the support device (13) comprises the cushion (19).

10. Mammography installation (10) according to one of the preceding claims, **characterised in that** the mammography installation (10) has a computing unit (20), wherein the integrated ultrasound transducer (16) is designed to capture a deformation of the support device (13) and the computing unit (20) is designed to reconstruct a radiographic image data record in accordance with the deformation of the support device (13).

11. Mammography installation (10) according to one of the preceding claims, **characterised in that** the mammography installation (10) has a further ultrasound transducer (21) in addition to the integrated ultrasound transducer (16), wherein the further ultrasound transducer (21) is arranged such that the breast locating region (17) is provided between the integrated ultrasound transducer (16) and the further ultrasound transducer (21).

12. Method for providing a radiographic image data record and an ultrasonic image data record of a breast (B) of a patient by means of a mammography installation (10), having the following steps:
- positioning the breast (10) of the patient in a breast locating region (17) of the mammography installation (10),
- capturing the radiographic image data record of the breast (B) of the patient by means of an X-ray emitter (14) and an integrated X-ray detector (15) of the mammography installation (10),
- capturing the ultrasonic image data record of the breast (B) of the patient by means of an evenly spread integrated ultrasound transducer (16) of a support device (13) of the mammography installation (10), wherein the support device (13) holds the breast (B) of the patient steady between the capturing of the image data records, and
- providing the radiographic image data record and the ultrasonic image data record,
**characterised in that**
the support device (13) and the evenly spread integrated ultrasound transducer (16) are designed to be deformable such that they can be moulded to the breast (B) of the patient, wherein the evenly spread integrated ultrasound transducer (16) is arranged on an elastic substrate.

13. Method according to claim 12, wherein the capturing of the ultrasonic image data record includes determining a deformation of the evenly spread integrated ultrasound transducer (16) and wherein the determining of the deformation of the evenly spread integrated ultrasound transducer (16) comprises the following steps:
- activating at least one ultrasound transducer element (22) of the evenly spread integrated ultrasound transducer (16) in such a way that ultrasound waves are emitted,
- capturing a propagation time of the ultrasound waves by means of the at least one ultrasound transducer element (22) and
- determining the deformation of the evenly spread integrated ultrasound transducer (16) on the basis of the propagation time of the ultrasound waves.

14. Method according to one of the claims 12 to 13, wherein the determining of the deformation of the evenly spread integrated ultrasound transducer (16) is effected using the captured radiographic image data record.

15. Method according to one of the claims 12 to 14, wherein a deformation of the support device (13) is captured by means of the evenly spread integrated ultrasound transducer (16) and wherein the radiographic image data record is reconstructed in accordance with the deformation of the support device (13).

## Revendications

1. Installation (10) de mammographie pour l'imagerie radiographique et sonographique d'une poitrine (B) d'une patiente, comportant
- un premier dispositif (11) de compression,
- un deuxième dispositif (12) de compression,
- un dispositif (13) d'appui,
- un émetteur (14) de rayons X,
- un détecteur (15) intégré de rayons X et
- un transducteur (16) intégré d'ultrasons, qui est plat,
- dans laquelle le deuxième dispositif (12) de compression a le détecteur (16) intégré de rayons X,
- dans laquelle le dispositif (13) d'appui est monté sur le premier dispositif (11) de compression ou sur le deuxième dispositif (12) de compression et
- dans laquelle, entre le dispositif (13) d'appui et le dispositif (11, 12) de compression, opposé au dispositif (12, 11) de compression sur lequel est monté le dispositif (13) d'appui, est prévue une région (17) d'enregistrement de la poitrine et la poitrine (B) de la patiente peut être mise en position dans la région (17) d'enregistrement de la poitrine,
- dans laquelle le dispositif (13) d'appui a le transducteur (16) intégré d'ultrasons et
- dans laquelle le transducteur (16) intégré d'ultrasons est divisé en direction de la région (17) d'enregistrement de la poitrine,
**caractérisée**
**en ce que** le dispositif (13) d'appui et le transducteur (16) intégré d'ultrasons sont constitués en étant déformables, de manière à pouvoir être mis à la forme de la poitrine (B) de la patiente, le transducteur (16) intégré d'ultrasons étant mis sur un substrat élastique.

2. Installation (10) de mammographie suivant la revendication 1, **caractérisée en ce que** l'installation (10) de mammographie a une unité (20) de calcul, l'installation (10) de mammographie étant constituée pour déterminer une déformation du transducteur (16) intégré d'ultrasons et l'unité (20) de calcul étant constituée pour reconstruire un jeu de données d'image sonographique en fonction de la déformation.

3. Installation (10) de mammographie suivant l'une des revendications précédentes, **caractérisée en ce que** le dispositif (13) d'appui a une membrane, le transducteur intégré d'ultrasons étant mis sur la membrane.

4. Installation (10) de mammographie suivant l'une des revendications précédentes, **caractérisée en ce que** le dispositif (11, 12) de compression, sur lequel le dispositif (13) d'appui est monté, est constitué sous la forme d'un cadre et le dispositif (13) d'appui est bloqué dans le cadre.

5. Installation (10) de mammographie suivant l'une des revendications précédentes, **caractérisée en ce que** le dispositif (13) d'appui a un coussin (19).

6. Installation (10) de mammographie suivant l'une des revendications précédentes, **caractérisée en ce que** le dispositif (13) d'appui a un premier côté tourné vers la région (17) d'enregistrement de la poitrine et un deuxième côté tourné vers le dispositif (11, 12) de compression, sur lequel est monté le dispositif (13) d'appui, le premier côté ayant le transducteur (16) intégré d'ultrasons.

7. Installation (10) de mammographie suivant les revendications 5 et 6, **caractérisée en ce que** le deuxième côté du dispositif (13) d'appui a le coussin (19).

8. Installation (10) de mammographie suivant l'une des revendications 1 à 5, **caractérisée en ce que** le dispositif (13) d'appui a un premier côté tourné vers la région (17) d'enregistrement de la poitrine et un deuxième côté tourné vers le dispositif (11, 12) de compression, sur lequel est monté le dispositif (13) d'appui, le deuxième côté du transducteur (16) intégré d'ultrasons ayant le deuxième côté.

9. Installation (10) de mammographie suivant les revendications 5 et 8, **caractérisée en ce que** le premier côté du dispositif (13) d'appui a le coussin (19).

10. Installation (10) de mammographie suivant l'une des revendications précédentes, **caractérisée en ce que** l'installation (10) de mammographie a une unité (20) de calcul, le transducteur (16) intégré d'ultrasons étant constitué pour détecter une déformation du dispositif (13) d'appui et l'unité (20) de calcul étant constituée pour reconstituer un jeu de données d'image radiographique en fonction de la déformation du dispositif (13) d'appui.

11. Installation (10) de mammographie suivant l'une des revendications précédentes, **caractérisée en ce que** l'installation (10) de mammographie a, en plus du transducteur (16) intégré d'ultrasons, un autre transducteur (21) d'ultrasons, l'autre transducteur (21) d'ultrasons étant disposé de manière à ce que la région (17) d'enregistrement de la poitrine soit prévue entre le transducteur (16) intégré d'ultrasons et l'autre transducteur (21) d'ultrasons.

12. Procédé pour disposer d'un jeu de données d'image radiographique et d'un jeu de données d'image sonographique d'une poitrine (B) d'une patiente, au moyen d'une installation (10) de mammographie, comprenant les stades suivants :
- on met la poitrine (10) de la patiente en position dans une région (17) d'enregistrement de la poitrine de l'installation (10) de mammographie,
- on relève le jeu de données d'image radiographique de la poitrine (B) de la patiente au moyen d'un émetteur (14) de rayons X et d'un détecteur (15) intégré de rayons X de l'installation (10) de mammographie,
- on relève le jeu de données d'image sonographique de la poitrine (B) de la patiente au moyen d'un transducteur (16) intégré d'ultrasons plat d'un dispositif (13) d'appui de l'installation (10) de mammographie, dans lequel, entre le relevé des jeux de données d'image, le dispositif (13) d'appui maintient immobilisée la poitrine (B) de la patiente, et
- on met à disposition le jeu de données d'image radiographique et le jeu de données d'image sonographique,
**caractérisé**
**en ce que** le dispositif (13) d'appui et le transducteur (16) intégré d'ultrasons sont constitués en étant déformables, de manière à pouvoir être mis à la forme de la poitrine (B) de la patiente, le transducteur (16) intégré d'ultrasons étant mis sur un substrat élastique.

13. Procédé suivant la revendication 12, dans lequel le relevé du jeu de données d'image sonographique comprend un détermination d'une déformation du transducteur (16) intégré d'ultrasons plat et dans lequel la détermination de la déformation du transducteur (16) intégré d'ultrasons plat comprend les stades suivants :
- on commande au moins un élément (22) du transducteur (16) intégré d'ultrasons plat, de manière à émettre des ondes ultrasonores,
- on relève un temps de parcours des ondes ultrasonores au moyen du au moins un élément (22) de transducteur d'ultrasons et
- on détermine la déformation du transducteur (16) intégré d'ultrasons plat en fonction du temps de parcours des ondes ultrasonores.

14. Procédé suivant l'une des revendications 12 à 13, dans lequel la détermination de la déformation du transducteur (16) intégré d'ultrasons plat s'effectue en utilisant le jeu de données d'image radiographique relevé.

15. Procédé suivant l'une des revendications 12 à 14, dans lequel on relève, au moyen du transducteur (16) intégré d'ultrasons plat, une déformation du dispositif (13) d'appui et dans lequel on reconstruit le jeu de données d'image radiographique en fonction de la déformation du dispositif (13) d'appui.
